# EUROPEAN PATENT APPLICATION

(11) **EP 4 321 086 A1**
(43) Date of publication of application: **14.02.2024**
(21) Application number: 23165228.0
(22) Date of filing: 29.03.2023
(51) Int. Cl.: A61B 5/00, A61N 5/06, H04R 1/10

(54) **NECKBAND SENSING DEVICE**

(30) Priority: 08.08.2022 TW 111129766; 28.03.2023 TW 112111810
(71) Applicant: Decentralized Biotechnology Intelligence Co., Ltd., Taipei 106021 (TW)
(72) Inventor: Chou, Yao-Sheng, 106021 Taipei (TW); Chou, Yen-Han, 106021 Taipei (TW)
(74) Representative: Zaboliene, Reda

(57) **Abstract**

The present invention discloses a neckband sensing device including a processor; a sensor is coupled to the processor, the sensor is selected form a blood pressure sensor, a heart rate sensor, an infrared sensor, a temperature sensor, an ultrasonic sensor, piezoelectric vibration sensor or the combination thereof. A light therapy device is coupled with the processor to emit therapy light.

## Description

### TECHNICAL FIELD

The present invention relates to a sensing device, more specifically, a neckband device having a sensing component.

### BACKGROUND

A headset audio device has earmuffs that constitute an airtight space to propagate sound for the listener. Another type audio devices, such as loudspeakers, are designed to transmit sound to a certain distance. Headphones and loudspeakers are similar in structure, but the difference lies in the power for vibrating the air. The power required by the earphone speaker is lower than the one of the loudspeakers for sound transmission.

The earphones are designed to output power of the speaker in the earmuff shells. Neckline-type earphones and neckband-type earphones enable wireless transmission between sound sources and the earphones, support stereo playback, noise reduction. The stereo effect of the traditional neckband-type earphones can not satisfy the user's demand for use in various scenarios due to the distance between the two speakers is too close.

Currently, the conventional earphone transmits the sound from the internal speaker, and the earphones are generally classified into in-earphone, on-earphone and over-earphone. No matter what type it is, the speakers are too close to the eardrum, it causes discomfort or harms ear soft tissue after a long-time usage, thereby resulting in hearing loss.

Under the conventional over-earphone design, the ears are covered by the earphone when it is employed. It will reduce the user's sensitivity to the surrounding things and cause accidents by ignoring the warnings. Furthermore, the general audio device is unlikely to be worn on the body due to its volume or weight, up to now, there is no effective way to solve the issue caused by the earphone.

What is required is to avoid above various shortcomings, and to achieve better solution for the conventional earphone.

### SUMMARY OF THE INVENTION

Based on the above-mentioned, the method of noise reduction for earphone has become an important work in many fields. For example, a database of noise reduction parameters or noise reduction levels of various noise sources are established to facilitate flexible adjustment of noise reduction levels, improve the performance of noise reduction to achieve the purpose of the present invention.

The purpose of the present invention is to provide a neckband sensing device including a processor; a sensor is coupled to the processor, the sensor is selected form a blood pressure sensor, a heart rate sensor, an infrared sensor, a temperature sensor, an ultrasonic sensor, piezoelectric vibration sensor or the combination thereof. A light therapy device is coupled with the processor to emit therapy light. For red light therapy: it operates at a wavelength of 630nm to 700nm. Blue light therapy: it operates at a wavelength of 446nm to 477nm, and infrared red-light therapy: it operates at a wavelength of 700nm to 1200nm.

The purpose of the present invention is to provide a neckband sensing device having noise reduction device and method for improving the performance of noise reduction, especially a device for detecting heart sound.

According to one aspect of the present invention, the mixed audio signal includes an inverse noise of ambient noise. When the input noise pattern/waveform signal (inverse noise) and the noise source enter the ear canal of human together, the inverted noise and the noise source will produce destructive interference because of mutual cancellation of the waveform, so as to achieve the performance of reducing the noise decibel value. By using the recursive method to limit the frequency step by step, the noise generated by the noise source can be effectively suppressed to improve the performance of noise reduction. It should be understood that the inverse noise can completely offset the noise of the noise source, and can also partially offset the noise of the noise source.

According to another aspect of the present invention, the present invention includes a noise reduction device to reduce a noise decibel value generated by the noise source.

A wearable sensing device includes a voice control device coupled to the processor for controlling functions by voice; a communication device is coupled to the processor for communicating with an external device.

According to yet another aspect of the present invention, a warning device is coupled to the processor, it can issue waring signal by buzzer sound or light emitting diode (LED) light.

### BRIEF DESCRIPTION OF THE DRAWINGS

Figure 1 shows the hanging and sensing audio device according to one embodiment of the present invention;
Figure 2 shows a functional diagram according to an embodiment of the present invention;

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

The present invention provides a wearable sensing device, for example a neckband sensing device, the present invention not only propagates high-quality stereo sound, but also reduces noise. The present invention also offers communication, intelligent voice control, heart rate sensing (heart rhythm monitoring), blood pressure sensing, infrared blood oxygen sensing, heating and other functions. In addition, the wearable audio device is combined with virtual reality (Virtual Reality; VR) and/or augmented reality (Augmented Reality; AR) to achieve an immersive feeling.

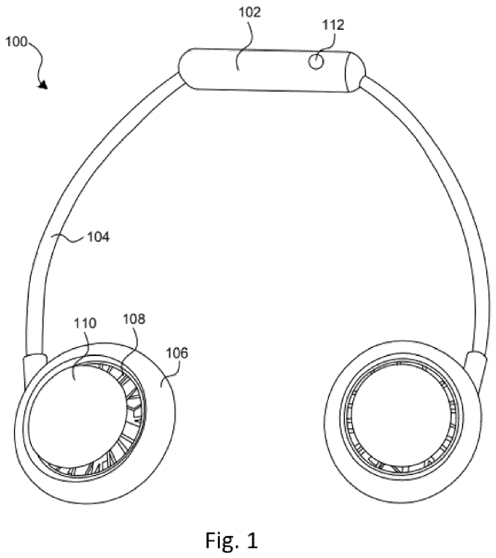

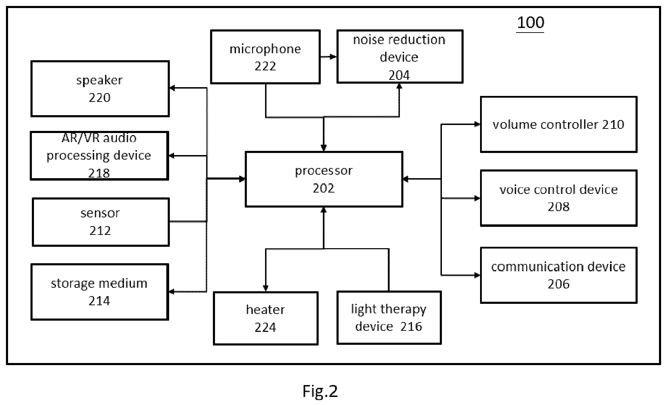

Referring to Fig. 1, it shows a schematic diagram of the embodiment of the neckband sensing device of the present invention. In this embodiment, the neckband sensing device 100 includes a neckband main body 102, two flexible connectors 104 and two optional speakers 106, the wherein one end of the connector 104 is extended and connected to the speakers 106, and the other end is connected to the neckband main body 102. The speaker 106 is located inside the shell to propagate sound outward. In addition, a baffle 110 is configurated on the upper part of speaker 106, and the baffle 110 is detachable. At least one outlet 108 is formed adjacent to the baffle 110. The sound is propagated from the outlet 108 to the surroundings when the speaker is active. The flexible connector 104 is a bendable member, thereby reducing the neckband sensing device volume, it is convenient to receive the device in a storage. In one embodiment, a battery is provided inside the neckband main body 102, and a power switch 112 is arranged for turning on or off the power. The other components of the neckband sensing device 100 maybe referred to figure 2.

As shown in the figure 2, it shows the functional block diagram of the neckband sensing device according to the embodiment of the present invention. In this embodiment, the neckband sensing device 200 is fit on the user's head, neck, shoulder by adjusting the flexible connectors 104. The neckband sensing device 200 includes a processor 202, a noise reduction device 204, a communication device 206, a voice control device 208, a volume controller 210, a sensor 212, a storage medium 214, an infrared detector 216, and AR/VR audio processing device 218, speakers 220, a microphone 222 and a heater 224. The processor 202 is coupled to the noise reduction device 204, the communication device 206, the voice control device 208, the volume controller 210, the sensor 212, the storage medium 214, the infrared detector 216, the AR/VR audio processing device 218, and the speaker 220, a microphone 220 and a heater 224 for controlling these elements. The noise reduction device 204 is employed for reducing noise generated by various noise sources. For example, the noise reduction device 204 employs a noise filter to generate the corresponding noise waveform signal for offsetting the noise signal generated by the noise source. The destructive interference is generated to reduce the noise decibel value (dB value) of the noise source. The purpose of noise reduction is therefore achieved.

Please refer to figure 2, the receiver 222 is used to receive the noise generated by the external noise source, and output the noise signal in an analog manner, the receiver 222 is, for example, a microphone. A noise patterns of various noise sources are stored in the noise pattern database in advance, and the various noise reduction parameter database is generated according to the noise waveform patterns. The noise reduction parameter database includes noise reduction level indexes and the corresponding noise reduction parameters. Namely, a noise reduction level is corresponded to a noise reduction parameter. For example, the noise reduction parameter database includes 32 a group of noise reduction level indexes, and a group of noise reduction parameters, preferably, the noise reduction level index 1 corresponds to parameter 1, and the noise reduction level index 2 corresponds to parameter 2, ... level index 32 corresponds to parameter 32, and so on. The noise reduction parameter database includes a set of parameters including a plurality of filter coefficients. The noise analog signal generated by the noise source received by the receiver 222 is identified via the noise waveform pattern identifier of the receiver 222. In another example, if there is no pre-stored noise pattern in the noise waveform database, a new noise pattern will be automatically created and stored for subsequent noise reduction. For example, the ambient noise is detected before the music is played in the audio device 200, and the noise is reduced during the play. The noise pattern is continuously updated (refresh) when the music is playback. In other words, the aforementioned situation is applied to the noise reduction in open spaces. The processor 102 selects a preset noise waveform corresponding to the received noise signal from the noise waveform database, and identify the received noise signal according to the noise waveform identifier. The processor 102 transmits the corresponding noise waveform information to the noise filter. In order to reduce the noise generated by the noise source, the noise filter eliminates the noise signal by the corresponding noise waveform signal, and the speaker outputs the processed signal after the corresponding noise waveform signal is selected by the processor 102. For one embodiment, the noise waveform database is received from a remote server or a remote device through the communication device 206. As an example, the noise waveform database can also be stored in the storage medium 214.

In one embodiment, the user of the audio device 200 may communicate with the user of another communication device (such as a smart phone, a tablet computer) through the communication device 206. For example, the communication device 206 communicates through a wireless network including Bluetooth, WLAN, Wifi, 3G, 4G, 5G and other wireless networks of various wireless specifications. In another embodiment, the audio device 200 is used as an audio output/input device of a communication device (such as a smart phone, a tablet computer), to phrase another word, the communication device may receive/send message from/to another user.

In one embodiment, the voice control device 208 manipulates the operation functions, such as controlling the volume, selecting and playing music tracks, etc., of the audio device 200 by the intelligent voice control via the received voice message.

In one embodiment, the voice control device 208 includes a voice recognition module, a natural language processing module, a dialog management module, and an intention judgment module. The voice recognition module is used for performing voice recognition processing on the receiving voice to obtain voice recognition information. The natural language processing module includes a semantic understanding module and a dialogue generation module. The semantic understanding is the ability of a machine to process the meaning and context behind real-world information. This semantic understanding module is used to perform semantic understanding processing on speech recognition information to obtain corresponding semantic understanding information, and input the semantic understanding information to the dialogue management module. The corresponding dialog management information is obtained by performing dialog management processing. The dialog management information is subsequently input into the intention judgment module to perform intention judgment processing to obtain corresponding intention judgment information. In addition, the voice control device 208 further includes a text-to-speech module. When the dialog management module generates a dialog management message, the dialog management message is additionally output to the dialog generating module for dialog generation processing to obtain a corresponding dialog generating message. Next, the dialog generated message is input to the text-to-speech module for text-to-speech processing to obtain the corresponding dialog voice message, and then the dialog voice message is output to the user through the audio playing device.

In one embodiment, the sound volume of the audio device 200 is adjustable through the volume controller 210 of the audio device 200. For example, the ambient noise is defined as normal, low and high ambient noise, and the output sound of the audio device 200 is also based on the level of the normal, low and high noise standard. For example, when the surrounding environment is at the level of normal ambient noise, the volume controller 210 correspondingly adjusts the sound of the audio device 200 to the normal audio sound level according to the normal ambient noise; when the ambient environment is at the low ambient noise level, then the volume controller 210 correspondingly adjusts the output sound of the audio device 200 to the low-audio sound level according to the low ambient noise. Conversely, when the audio device 200 is at the high ambient noise environment, the volume controller 210 will correspondingly adjusts the sound level of the audio device 200 to high level according to the high ambient noise.

In one embodiment, the audio device 200 includes a heart sound sensing device, it is beneficial to the medical application. In this embodiment, the sensor 212 is built in the audio device 200, and the sensor 212 includes a heart rate sensor, a blood pressure sensor, an infrared blood (oxygen) sensor, ultrasonic device, piezoelectric vibration sensor, temperature sensor...etc. The present invention includes a light therapy device coupled to the process. It can help improve seasonal depression disorder if the user sits close to a special light source for at least 30 minutes. The treatment also be effective for major depressive disorder and that occurs during or after pregnancy. Red light therapy: It operates at a wavelength of 630nm to 700nm, it is visible light spectrum. It may promote wound healing and improve hair growth in those with alopecia, relieve symptoms of arthritis. Blue light therapy: It operates at a wavelength of 446nm to 477nm, it is visible light spectrum. It may be used to tackle problem areas on or just beneath the skin. It may help to manage symptoms of psoriasis. Infrared red-light therapy: It operates at a wavelength of 700nm to 1200nm, it is invisible light spectrum. It may penetrate deep into the skin, capable of reaching muscles and nerves. It may help cell regeneration and repair, to improve circulation, promote healing and relieve chronic pain. The heart rate sensor is used to monitor and detect the heart rate. The blood pressure sensor is used to sense the blood pressure value, and the infrared blood oxygen sensor is used to measure the blood oxygen saturation level. If the sensor 212 is closely attached to the body near the heart, the heart rate sensor may detect the user's heart beats, thereby monitoring the user's heart rate. For example, the heart rate sensor receives heart sound signals that meet the predetermined frequency range, and the processor subsequently performs the calculation process to obtain the peak intensity v. time relationship data. The device records the signal-to-noise ratio value of each measurement position, and compares each the detected signal-to-noise ratio value, thereby determining the optimal measurement position for detecting the user's heart rate, and output the optimal measurement heart rate. For example, if one of these heart rate data, blood pressure value, blood oxygen saturation level is abnormal, a warning signal will be issued, and the abnormal data will be transmitted to an external device through the communication device 206. In another example, a buzzer sound or light emitting diode (LED) light may act as the warning signal to indicate an abnormal situation. In another example, the sensor 212 includes an ultrasonic sensor for detecting sound. In another example, the sensor 212 can be attached on the human body by a patch, and coupled to the neckband main body 102 by wire or wireless. In another example, the sensor 212 is disposed on the clothing by a storage bag or detachable accessory, alternatively, through a connecting piece, such as buckles, velcro or zippers. Take the velcro as an illustration, the sensor 212 is attached to the clothes by using velcro which is composed of two fabrics, typically. One surface includes a ring structure (hair surface), and the other one has a hook structure (hook surface). When the two pieces are engaged, the two fabrics combine together, thereby fastening temporarily. The combined pieces maybe separated by force. One fabric of the velcro is sewn on the clothes, and the sensor 212 is configurated on the other fabric.

In one embodiment, the storage medium 214 is employed to store music data, other audio data or application software. The storage medium 214 includes, for example, a memory and a computer-readable medium. The memory stores software or programs that can be run by the processor. Memory includes non-permanent memory, random access memory (RAM) and/or nonvolatile memory, such as read only memory (ROM) or flash memory (Flash RAM). Storage media include, but are not limited to: phase change memory (PRAM), static random access memory (SRAM), dynamic random access memory (DRAM), other types of random access memory (RAM) or read-only memory A memory (ROM) can be used to store information that can be accessed by a computing device.

In one embodiment, the sensor 212 of the wearable sensing device 100 includes an infrared sensor. The infrared sensor can continuously sense the body temperature of the living body and generate a plurality of body temperature sensing signals. It makes the user feel comfortable by using the sensor 212 to measure the body temperature without touching the body. When the wearable sensing device 100 determines that the user's body temperature is greater or lower than predetermined temperature values, it sends a warning message to an external electronic device to indicate the user's physical condition by the external electronic device. In another example, the infrared sensor 212 may regularly detect the blood sugar level of the user, and it transmits the blood sugar level to the processor 202, followed by determining whether the blood sugar level is higher or lower than a predetermined value. When the wearable sensing device 100 determines that the blood sugar level of the user is higher or lower than the pre-set value, it sends a warning message to the external electronic device. The heater 224 provides hot source to enable warm keeping function.

In one embodiment, the neckband sensing device 100 includes an AR/VR audio processing device 218, which can play virtual reality/augmented reality (AR/VR) audio, it allows the user with immersive experiences in virtual reality or augmented reality. Virtual reality (also known as artificial environment) uses computer simulation to generate a three-dimensional virtual world to provide users with simulations of vision, hearing, touch and other senses, allowing users to experience real-time, observe things in three-dimensional space without limitation. The AR/VR audio processing device 218 is utilized to process the streaming data downloaded from the external device (such as smart phone) to play the video/audio content of virtual reality. The AR/VR audio processing device 218 plays the sound of the virtual reality, and displays the virtual reality image content on the display of the external device. Since the neckband sensing device 100 is wearable on the user's body, the sound sources are closer to the user, so that the user can experience a more immersive experience.

In one embodiment, the neckband sensing device 100 is configured to communicate with an external device, and the external device may be an external computing device, computing system, mobile device (smart phone, tablet computer, smartwatches, etc.), or other types of electronic devices.

External devices include computing cores, user interfaces, internet interfaces, wireless communication transceivers, and storage devices. The user interface includes one or more input devices (e.g., keyboard, touch screen, voice input device.. etc.), one or more audio output devices (e.g. speakers.. etc.) and/or one or more visual output devices (eg, video graphics displays, touch screens..etc.). The internet interface includes one or more networked devices (eg, wireless local area network (WLAN) devices, wired LAN devices, wireless wide area network (WWAN) devices ... , etc.). Storage devices include flash memory devices, one or more hard disk drives, one or more solid state storage devices and/or cloud storage.

A computing core includes a processor and other computing core components. Other computing core components include a video graphics processing unit, a memory controller, main memory (eg, RAM), one or more input/output (I/O) device interface modules, input/output (I/O) interfaces, Input/Output (I/O) controller, peripheral device interface, one or more USB interface modules, one or more network interface modules, one or more memory interface modules and/or one or more Peripheral device interface module.

The external device processes the data transmitted by the communication component 206 to generate various results.

As will be understood by persons skilled in the art, the foregoing preferred embodiment of the present invention illustrates the present invention rather than limiting the present invention. Having described the invention in connection with a preferred embodiment, modifications will be suggested to those skilled in the art. Thus, the invention is not to be limited to this embodiment, but rather the invention is intended to cover various modifications and similar arrangements included within the spirit and scope of the appended claims, the scope of which should be accorded the broadest interpretation, thereby encompassing all such modifications and similar structures. While the preferred embodiment of the invention has been illustrated and described, it will be appreciated that various changes can be made without departing from the spirit and scope of the invention.

## Claims

1. A neckband sensing device (100) including a processor (202), wherein the improvement comprising a light therapy device (216) coupled to said processor (202); and a sensor (212) coupled to said processor (212), wherein said sensor (212) is selected from the group of an infrared sensor, a blood pressure sensor, heart rate sensor, a temperature sensor, an ultrasonic device, a piezoelectric vibration sensor or the combination thereof.

2. The device of claim 1, wherein a wave length of a light emitted by said light therapy device (216) is 446nm-477nm.

3. The device of claim 1, wherein a wave length of a light emitted by said light therapy device (216) is 630nm-700nm.

4. The device of claim 1, wherein a wave length of a light emitted by said light therapy device (216) is 700nm-1200nm.

5. The device of claim 1, wherein said neckband sensing device (100) includes speakers (220) and a sound receiver (222) coupled to said processor (202).

6. The device of claim 5, wherein said neckband sensing device (100) includes a voice control device (208) coupled to said processor (202).

7. The device of claim 5, wherein said neckband sensing device (100) includes a communication device (206) coupled to said processor (202).

8. The device of claim 7, wherein said neckband sensing device (100) includes an AR/VR audio device (218) is coupled to said processor (202).

9. The device of claim 5, wherein a noise reduction device (204) is coupled to said processor (202) to reduce noise.

10. The device of claim 9, wherein a noise pattern recognizer is coupled to said processor (202) for identifying a noise received by said sound receiver.

11. The device of claim 10, wherein a noise pattern database is coupled to said processor (202), wherein said noise pattern database includes a plurality of preset noise pattern, and a plurality of noise reduction parameters.

12. The device of claim 11, wherein a noise filter is coupled to said processor (202), destructively interfering said noise with a selected noise pattern.

13. The device of claim 1, wherein said infrared sensor detects a blood oxygen saturation level and a blood sugar level.

14. The device of claim 1, wherein a heater (224) coupled to said processor (202).

15. The device of claim 1, wherein said neckband sensing device (100) includes a neckband main body (102), two flexible connectors (104) connected with said neckband main body (102).
